# EUROPEAN PATENT APPLICATION

(11) **EP 4 803 611 A1**
(43) Date of publication of application: **09.09.2026**
(21) Application number: 24885549.6
(22) Date of filing: 23.10.2024
(51) Int. Cl.: C12N 5/075, A01K 67/02, C12N 5/073

(54) **COMPOSITION FOR TREATING IMMATURE EGG AND/OR EMBRYO (FERTILIZED EGG)**

(30) Priority: 31.10.2023 JP 2023186579
(71) Applicant: University Public Corporation Osaka, Osaka-shi, Osaka 5360025 (JP); Rohto Pharmaceutical Co., Ltd., Osaka-shi, Osaka 544-8666 (JP)
(72) Inventor: HASHIMOTO, Shu, Sakai-shi, Osaka 599-8531 (JP); KITAJI, Hideki, Sakai-shi, Osaka 599-8531 (JP); TAKIJIRI, Takashi, Osaka-shi, Osaka 544-8666 (JP)
(74) Representative: Mewburn Ellis LLP
(86) International application number: PCT/JP2024/037680
(87) International publication number: WO 2025/094776

(57) **Abstract**

The purpose of the present invention is to provide a culture medium for immature eggs that is excellent in quality and safety and that is capable of efficiently maturing immature eggs with respect to immature eggs for vitro maturation-in vitro fertilization-embryo transfer method (IVM-IVF). The present invention is a composition that is for treating immature eggs and/or embryos (fertilized eggs) and that contains a serum-free mesenchymal stem cell culture medium. The treatment composition is suitably used as an immature egg culture agent, an embryo (fertilized egg) culture agent, an agent for assisting implantation, an embryo transfer solution or an implantation rate improver.

## Description

### TECHNICAL FIELD

The present invention relates to a composition for treating immature eggs and/or embryos (fertilized eggs).

### BACKGROUND ART

"Infertility" refers to a condition in which a healthy man and a healthy woman who wish to conceive have sexual intercourse without contraception but the woman does not become pregnant for a certain period of time. The Japan Society of Obstetrics and Gynecology defines the "certain period of time" as "one year in general." It is said that about one in ten couples is infertile, but in recent years, since the age at which people consider pregnancy has been increasing, and it is known that the pregnancy becomes more difficult with the age in both men and women, it is also said that this proportion is higher than one in ten.

Examples of causes of female infertility include ovulatory factors such as ovulation disorders, tubal factors such as obstruction of the fallopian tubes, stenosis of the fallopian tubes, and adhesion of the fallopian tubes, uterine factors such as uterine fibroids and endometrial polyps, cervical factors such as cervicitis and abnormal mucus secretion from the cervical canal, and immune factors such as antisperm antibodies. In general, it is considered that the tubal disorders account for the highest frequency among the causes of the female infertility.

Treatment of the infertility is carried out by selecting the optimal treatment method according to the cause. Examples of major treatment methods include timed intercourse method, ovulation induction method, artificial insemination, and further assisted reproductive technologies such as in vitro fertilization and intracytoplasmic sperm injection.

In vitro fertilization-embryo transfer (IVF-ET) method includes a fresh embryo transfer method and a frozen-thawed embryo transfer method. In the fresh embryo transfer method, embryo transfer is carried out in the same cycle in which the eggs are retrieved under ovarian stimulation or in a natural cycle. In the frozen-thawed embryo transfer method, after the egg retrieval, the fertilized eggs or the embryos are once cryopreserved, and the embryo transfer is carried out in a subsequent cycle.

In vitro maturation-in vitro fertilization-embryo transfer (IVM-IVF) method is a method in which immature eggs is collected from the ovary of a patient who does not undergo ovarian stimulation, or to whom only a very small amount of ovulation inducing agent has been administered, matured in vitro (in vitro maturation: IVM), and then the matured ova are fertilized by the intracytoplasmic sperm injection or standard insemination, followed by transfer of the developed fertilized eggs into the uterus. This method is selected in a case in which the ovarian follicle does not mature normally even when ovarian stimulation with the ovulation inducing agent is carried out, in a case in which the ova must be collected at an immature stage in order to avoid ovarian hyperstimulation syndrome (OHSS), which may occur due to an excessive ovarian response to the ovulation inducing agent, in a patient at a high risk of developing the OHSS, or in a similar case. In addition, it can also be used in a case in which, for some reason, there is a problem with the maturation of the eggs under a normal process and the eggs cannot develop into mature eggs which are capable of sufficiently accepting the sperm.

Unlike the IVF-ET, the IVM-IVF has the advantage of reducing the physical burden caused by the administration of an agent and reducing the economic burden, because the ovarian stimulation is hardly carried out. In addition, since it can also be used for cryopreserved cells, it is considered that the range of treatment options for patients can be expanded. Furthermore, it may be applicable to infertility treatment for a patient whose immature eggs do not mature in the ovary.

However, in the IVM-IVF, it is a problem that with the existing methods the maturation efficiency of immature eggs and the rate of treatment success remain low. In addition, many of the culture media currently used for the culture of immature eggs contain serum (see Non Patent Literature 1). It is said that the serum is extremely important as a source of growth factors, adhesion factors, hormones, lipids, and minerals that are necessary for culturing cells in a basal culture medium. However, the serum contains many components, including unknown substances. In addition, since the components differ depending on the serum lot, the performance tends to vary, and it is difficult to obtain a serum-containing culture medium of stable quality. Moreover, since the serum is a biologically derived material, there are risks of infection and allergy, and there are also concerns regarding safety. Accordingly, there is a need for the development of a culture medium for immature eggs that has the stable quality and the high safety, as well as for the development of a culture method capable of improving the maturation efficiency of the immature eggs.

### CITATION LIST

### Non Patent Literature

Non Patent Literature 1: J. Mamm. Ova Res., 1998, vol. 15, 109-112

### SUMMARY OF INVENTION

### Technical Problem

In order to carry out the IVM-IVF, it is necessary to mature immature eggs in vitro to a stage at which they can be used for the in vitro fertilization. Under the circumstances described above, it is an object of the present invention to provide a culture medium for immature eggs that has the stable quality and the high safety. In addition, it is another object of the present invention to provide an excellent method for maturing immature eggs and an infertility treatment that can bring more treatment opportunities to a patient by efficiently maturing the immature eggs in vitro, and that can further enhance the development of the embryos (the fertilized eggs) and the implantation efficiency at the time of the embryo transfer.

### Solution to Problem

As a result of diligent studies conducted to achieve the objects described above, the present inventors have found that a serum-free medium for mesenchymal stem (stromal) cells (MSC) can mature immature eggs, and thus completed the present invention. According to the present invention, immature eggs can be efficiently matured, the development of embryos (fertilized eggs) can be promoted, and the implantation rate can be increased. In detail, the gist of the present invention is as follows.
[1] A composition for treating immature eggs and/or embryos (fertilized eggs) containing a serum-free mesenchymal stem cell culture medium.
[2] The composition for treating according to [1], wherein the composition is used as an agent for culturing immature eggs, an agent for culturing embryos (fertilized eggs), an agent for assisting implantation, an embryo transfer solution, or an agent for improving an implantation rate.
[3] The composition for treating according to [1] or [2], wherein the serum-free mesenchymal stem cell culture medium contains EGF, bFGF, albumin, transferrin, and insulin.

### Advantageous Effects of Invention

According to the composition for treating immature eggs and/or embryos (fertilized eggs) of the present invention, immature eggs can be efficiently matured, the development of embryos can be promoted, and the implantation rate can be increased in the IVM-IVF.

### BRIEF DESCRIPTION OF DRAWING

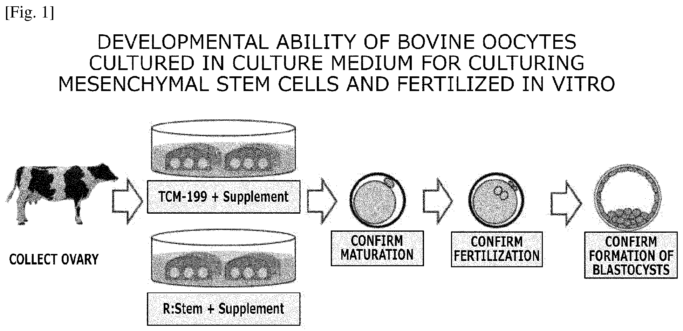

[Fig. 1] Fig. 1 is a diagram illustrating a scheme of a test for evaluating developmental ability of mature eggs after in vitro maturation and in vitro fertilization.

### DESCRIPTION OF EMBODIMENTS

Hereinafter, the composition for treating immature eggs and/or embryos (fertilized eggs) of the present invention will be described in detail. In addition, an agent for culturing immature eggs, an agent for culturing embryos (fertilized eggs), an agent for promoting maturation of immature eggs, an agent for promoting formation of embryos (fertilized eggs), an agent for promoting formation of blastocysts, an agent for assisting implantation, an embryo transfer solution, and an agent for improving an implantation rate of the present invention will also be described.

### [Composition for Treating Immature Eggs and/or Embryos (Fertilized Eggs)]

The composition for treating immature eggs and/or embryos (fertilized eggs) according to the present invention (hereinafter, also simply referred to as the "composition for treating") is characterized by containing a mesenchymal stem cell culture medium. According to the composition for treating of the present invention, since it contains the mesenchymal stem cell culture medium, immature eggs can be efficiently matured, the development of embryos (fertilized eggs) can be promoted, and the implantation rate can be increased, when the immature eggs are cultured in the in vitro maturation-in vitro fertilization-embryo transfer (IVM-IVF). The composition for treating of the present invention is suitably used for, in addition to the culture of immature eggs, purposes such as culture of embryos (fertilized eggs), transfer of embryos (fertilized eggs), assistance of implantation of embryos (fertilized eggs), and improvement of an implantation rate of embryos (fertilized eggs), and can be used for improvement of the success rate and the efficiency of artificial insemination and in vitro fertilization in humans, reproduction of livestock (improvement of the success rate and the efficiency of production and transfer of eggs fertilized in vitro), breeding and species conservation of animals (for example, conservation of endangered species, maintenance or crossing of pet lineages), treatment or amelioration of various diseases in which impaired oocyte maturation is a primary or secondary factor, and the like.

### (Mesenchymal Stem Cell Culture Medium)

In the present invention, the mesenchymal stem cell culture medium is a culture medium used for culturing mesenchymal stem cells.

In the present invention, the term "mesenchymal stem cell" refers to cells having the ability to differentiate into one or more types of cells belonging to mesenchymal tissues (such as bone cells, cardiac myocytes, chondrocytes, tendon cells, and adipocytes), and capable of proliferating while maintaining such an ability. As used in the context of the present invention, the term "mesenchymal stem cell" means the same cells as stromal cells, and the two are not particularly distinguished from each other.

The culture medium used for culturing mesenchymal stem cells in the present invention is not particularly limited as long as it is a culture medium capable of culturing the mesenchymal stem cells while maintaining them in a favorable state, but is preferably, for example, a culture medium capable of proliferating human mesenchymal stem cells while maintaining them with the ability to differentiate into bone cells, chondrocytes, and adipocytes.

The mesenchymal stem cell culture medium of the present invention may be prepared by adding one or more serum substitutes such as albumin, transferrin, fatty acids, insulin, sodium selenite, cholesterol, collagen precursors, trace elements, 2-mercaptoethanol, and 3'-thiolglycerol to a basal culture medium. In addition, such a culture medium may be supplemented with a substance including amino acids such as glutamine, sugars such as glucose, metal salts such as sodium chloride and magnesium sulfate, trace metals such as selenium, lipids such as cholesterol and unsaturated fatty acids, vitamins such as pantothenic acid, proteins such as growth factors, proliferation factors, and cytokines, polysaccharides, low-molecular compounds, antibiotics, antioxidants, pyruvic acid, buffers, and inorganic salts, if necessary.

Examples of the basal culture medium include the IMDM culture medium, the Medium 199 culture medium, the Eagle's Minimum Essential Medium (EMEM) culture medium, the αMEM culture medium, the Dulbecco's modified Eagle's Medium (DMEM) culture medium, the Ham's F12 culture medium, the RPMI 1640 culture medium, the Fischer's culture medium, the MCDB201 culture medium, and mixed culture media thereof.

From the viewpoint of being used for treatment of immature eggs, the mesenchymal stem cell culture medium contained in the composition for treating of the present invention is preferably a culture medium that does not contain xenogeneic components such as serum (a xeno-free culture medium). Examples of such a culture medium include those provided as culture media prepared in advance for mesenchymal stem cells (stromal cells), such as the Mesenchymal Stem Cell Growth Medium 2 (Ready-to-use) (manufactured by PromoCell), the Mesenchymal Stem Cell Growth Medium XF (Ready-to-use) (manufactured by PromoCell), the MSCGM BulletKit(TM), the MSCGM(TM) Mesenchymal Stem Cell Growth Medium BulletKit(TM) (manufactured by Lonza), a xeno-free culture medium for human mesenchymal stem cells (the MSC NutriStem (R) XF, manufactured by Biological Industries), the MesenCult-ACF Plus (manufactured by Veritas), the StemXVivo(TM) Serum-Free Human MSC Expansion Media (manufactured by R&D Systems and Corning), a serum-free culture medium for adipose-derived stem cells (the KBM ADSC-4, manufactured by Kohjin Bio), and a serum-free culture medium for mesenchymal stem cells (R: STEM Medium for hMSC High Growth, manufactured by Rohto).

Examples of the fatty acids include, but are not limited to, linoleic acid, oleic acid, linolenic acid, arachidonic acid, myristic acid, palmitoleic acid, palmitic acid, and stearic acid. Examples of the lipids include, but are not limited to, phosphatidylserine, phosphatidylethanolamine, and phosphatidylcholine. The amino acids include, but are not limited to, for example, L-alanine, L-arginine, L-aspartic acid, L-asparagine, L-cysteine, L-cystine, L-glutamic acid, L-glutamine, and L-glycine. Examples of the proteins include, but are not limited to, ecotin, reduced glutathione, fibronectin, and β2-microglobulin. Examples of the polysaccharides include, but are not limited to, glycosaminoglycans, among which hyaluronic acid, heparan sulfate are particularly exemplified. Examples of the growth factors include, but are not limited to, epidermal growth factor (EGF), basic fibroblast growth factor (bFGF), platelet-derived growth factor (PDGF), transforming growth factor beta (TGF-β), hepatocyte growth factor (HGF), connective tissue growth factor (CTGF), and vascular endothelial growth factor (VEGF).

In addition to the mesenchymal stem cell culture medium, the composition for treating of the present invention may contain other components within a range that does not impair the effects of the present invention. Examples of other components include protective agents such as dimethyl sulfoxide (DMSO) and serum albumin, antibiotics, carriers, excipients, disintegrants, buffers, emulsifying agents, stabilizing agents, preservative agents, antiseptic agents, and physiological saline.

The species to which the composition for treating of the present invention is applied may be any mammal, and examples thereof include humans, horses, cattle, sheep, pigs, dogs, cats, rabbits, mice, rats, and rare animals, and preferred examples are humans, cattle, dogs, and cats.

The composition for treating of the present invention is used as a liquid that directly contacts immature eggs or embryos (fertilized eggs), such as an agent for culturing immature eggs, an agent for culturing embryos (fertilized eggs), an agent for promoting maturation of immature eggs, an agent for promoting formation of embryos (fertilized eggs), an agent for promoting formation of blastocysts, an agent for assisting implantation, an embryo transfer solution, and an agent for improving an implantation rate.

The agent for culturing immature eggs is a composition for maturing in vitro eggs collected in an immature state to a state in which it can be fertilized with sperm. It is also referred to as an agent for promoting maturation of immature eggs. It may be in the form of a culture solution (culture medium) used for culturing immature eggs, or may be in a form added to and used with the above culture solution (culture medium). The agent for culturing embryos (fertilized eggs) is a liquid for culturing in vitro fertilized eggs to a stage at which they can be transferred into the uterus, such as the blastocyst stage. It is also referred to as an agent for promoting formation of embryos (fertilized eggs) or an agent for promoting formation of blastocysts. The agent for assisting implantation is a preparation for assisting "implantation," in which fertilized eggs come into contact with and become established in the endometrium, so that it occurs successfully. The embryo transfer solution is a solution in which embryos (fertilized eggs) are placed in the "embryo transfer," in which the embryos (the fertilized eggs) are returned to the uterus after being grown to some extent. The agent for improving an implantation rate is a preparation for improving the implantation rate of embryos (fertilized eggs).

The composition for treating of the present invention can be prepared by mixing the mesenchymal stem cell culture medium described above with other necessary components in accordance with an ordinary method.

The present invention also encompasses a method for promoting the maturation of immature eggs, a method for promoting the formation of embryos (fertilized eggs), a method for promoting the formation of blastocysts, and a method for improving the implantation rate of fertilized eggs, that are in vitro methods, by using the composition for treating of the present invention as described above.

The method for promoting the maturation of immature eggs according to the present invention is a method including a step of culturing immature eggs collected from the ovary in the composition for treating (the culture medium) of the present invention as described above for from two hours to 90 hours, preferably from five hours to 60 hours, and more preferably from 10 hours to 48 hours. According to this method, the maturation rate of the oocytes can be improved, and the formation of the embryos (the fertilized eggs) and the formation of the blastocysts can be promoted, as compared with a conventional method.

The method for promoting the formation of embryos (fertilized eggs) and the method for promoting the formation of blastocysts according to the present invention include a step of fertilizing in vitro the oocytes matured using the composition for treating of the present invention with sperm, and then further culturing them using the composition for treating of the present invention for from one to 21 days, preferably from two to 14 days, and more preferably from four to eight days. According to this method using the composition for treating of the present invention, the formation of the embryos (the fertilized eggs) and the formation of the blastocysts can be promoted.

The method for improving the implantation rate of fertilized eggs according to the present invention is a method including, in addition to the method for promoting the maturation of immature eggs, the method for promoting the formation of embryos (fertilized eggs), and the method for promoting the formation of blastocysts, a step of treating the embryos (the fertilized eggs) with the composition for treating of the present invention in the embryo transfer in which the embryos (the fertilized eggs) are transferred into the uterus of a recipient. According to this method using the composition for treating of the present invention, the implantation rate of the fertilized eggs can be improved.

### EXAMPLE

The present invention will be described in detail below with reference to an Example and a test example, but the present invention is not to be limited by such an Example or the like.

### [Example: Examination of Developmental Ability of Immature Eggs after In Vitro Maturation and In Vitro Fertilization]

The test was conducted in accordance with the scheme illustrated in Fig. 1. Specifically, immature ova were collected from the ovaries of cattle (Japanese Black, Holstein, and a crossbred thereof), and the immature ova were cultured for 21 hours in the R:STEM culture medium (R:STEM Medium for hMSC High Growth; with the Confirmation of Eligibility for Materials of Regenerative Medical Products (PMDA/CPE Notification No. 492); Application for Registration of the Drug Master File is to be filed; a serum-free culture medium for mesenchymal stem cells, containing EGF, bFGF, albumin, transferrin, and insulin; manufactured by Rohto) supplemented with 1 µg/ml of β-estradiol (E-8875; Merck Millipore Co.) and 0.02 mg/ml of FSH (Antrin; Kyoritsu Tokyo, Japan), and the maturation rates of the ova were examined. In addition, as a control, the immature eggs were cultured for 21 hours in the TCM199 culture medium (Gibco, Grand Island, NY) supplemented with 1 µg/ml of β-estradiol (E-8875; Merck Millipore Co.) and 0.02 mg/ml of FSH (Antrin; Kyoritsu Tokyo, Japan). After overnight fixation of the ova with Carnoy's fixative (ethanol 3: acetic acid 1), chromosomes were stained by aceto-orcein staining. Under a phase-contrast microscope (x 400), the eggs showing a metaphase II image and release of the first polar body were determined to be mature eggs, and the maturation rate was measured. As a result, the maturation rates of the oocytes matured in the R:STEM culture medium and the TCM-199 culture medium were 86.3% and 77.9%, respectively.

Next, the eggs subjected to the maturation culture described above were fertilized in vitro with ejaculated sperm collected from cattle (Holstein), and then the obtained fertilized eggs were subjected to embryo culture using the SOFaa culture medium, and the oocytes matured in the R:STEM culture medium and the TCM-199 culture medium were evaluated for a normal fertilization rate and a cleavage rate. Regarding the normal fertilization rate, similarly to the measurement of the maturation rate, the oocytes were subjected to fixation with the Carnoy's fixative and the evaluation was carried out under the phase-contrast microscope. Oocytes having one male pronucleus and one female pronucleus and further having released two polar bodies were determined to be normally fertilized eggs. The cleavage rate was evaluated under a stereomicroscope (x 40) on the day 7 after the insemination, while determining oocytes undergoing cell division as cleaved eggs. As a result, the normal fertilization rates of the oocytes matured in the R:STEM culture medium and the TCM-199 culture medium were 59.0% and 47.6%, respectively, and the cleavage rates thereof were 97.5% and 91.7%, respectively.

In addition, blastocysts produced after seven days from the oocytes matured in the R:STEM culture medium and the TCM-199 culture medium were fixed and stained for 120 minutes at 4°C with a 100% ethanol solution containing 25 µg/ml of Hoechst33342 (Dojindo), and then the cell numbers were counted using a confocal microscope (CV1000, Yokogawa Electric Corporation). The cell numbers were 188.9 and 75.6, respectively, and the numbers of blastocyst cells produced after eight days were 143.3 and 128.9, respectively.

From the above results, it was clarified that, by culturing immature ova in the R:STEM culture medium, the ova matured efficiently, and further the maturation of the fertilized eggs was also promoted. Since culturing immature ova in the R:STEM culture medium also promotes the maturation of the fertilized eggs, it is considered that this also contributes to the improvement of the implantation rate of the embryos (the fertilized eggs).

### INDUSTRIAL APPLICABILITY

According to the composition for treating immature eggs and/or embryos (fertilized eggs) of the present invention, immature eggs can be efficiently matured, the development of embryos can be promoted, and the implantation rate can be increased in the IVM-IVF. The composition for treating of the present invention is suitably used for purposes such as culture of immature eggs, culture of embryos (fertilized eggs), transfer of embryos (fertilized eggs), assistance of implantation of embryos (fertilized eggs), and improvement of an implantation rate of embryos (fertilized eggs), and can be used for improvement of the success rate and the efficiency of artificial insemination and in vitro fertilization in humans, reproduction of livestock (improvement of the success rate and the efficiency of production and transfer of eggs fertilized in vitro), breeding and species conservation of animals (for example, conservation of endangered species, maintenance or crossing of pet lineages), treatment or amelioration of various diseases in which impaired oocyte maturation is a primary or secondary factor, and for other applications.

## Claims

1. A composition for treating immature eggs and/or embryos (fertilized eggs) comprising a serum-free mesenchymal stem cell culture medium.

2. The composition for treating according to Claim 1, wherein the composition is used as an agent for culturing immature eggs, an agent for culturing embryos (fertilized eggs), an agent for assisting implantation, an embryo transfer solution, or an agent for improving an implantation rate.

3. The composition for treating according to Claim 1 or 2, wherein the serum-free mesenchymal stem cell culture medium comprises EGF, bFGF, albumin, transferrin, and insulin.
